# FASCICULE DE BREVET EUROPEEN

(11) **EP 4 312 894 B1**
(45) Date de publication et mention de la délivrance du brevet: **04.12.2024**
(21) Numéro de dépôt: 22717857.1
(22) Date de dépôt: 24.03.2022
(51) Int. Cl.: A61F 2/54, B29C 64/386, B33Y 50/00, B33Y 70/00, B33Y 80/00

(54) **DISPOSITIF DE TRAITEMENT DE DONNÉES POUR LA GÉNÉRATION DE MICROSTRUCTURES A PROPRIÉTÉS DÉFORMABLES COMMANDABLES**
DATENVERARBEITUNGSVORRICHTUNG ZUR ERZEUGUNG VON MIKROSTRUKTUREN MIT STEUERBAREN VERFORMBAREN EIGENSCHAFTEN
DATA PROCESSING DEVICE FOR THE GENERATION OF MICROSTRUCTURES WITH CONTROLLABLE DEFORMABLE PROPERTIES

(30) Priorité: 24.03.2021 FR 2102932
(43) Date de publication de la demande: 07.02.2024
(73) Titulaire: Institut National de Recherche en Informatique et en Automatique, 78150 Le Chesnay (FR)
(72) Inventeur: LEFEBVRE, Sylvain, 78150 Le Chesnay (FR); MARTINEZ, Jonas, 78150 Le Chesnay (FR); KENZARI, Samuel, 78150 Le Chesnay (FR); ANDRE, Jean-Claude, 78150 Le Chesnay (FR); ETIENNE, Jimmy, 78150 Le Chesnay (FR)
(74) Mandataire: Vidon Brevets & Stratégie
(86) Numéro de dépôt international: PCT/FR2022/050553
(87) Numéro de publication internationale: WO 2022/200742

(56) Documents cités:
- FR-A1- 3 049 213
- US-A1- 2017 049 583
- US-A1- 2017 120 535
- US-A1- 2020 089 195
- US-A1- 2020 316 847

## Description

L'invention concerne le domaine de la fabrication additive. Plus particulièrement, elle concerne le domaine de la fabrication additive de structures et de microstructures à propriétés déformables.

Le document US 2017/120535 A1, considéré comme l'état de l'art le plus proche, divulgue un dispositif pour fabrication additive, comprenant une mémoire agencée pour recevoir des données de forme d'objet à fabriquer et des données d'actionneurs pour l'objet à fabriquer, lesdites données d'actionneurs définissant des actionneurs configurés pour permettre une déformation prédéfinie et commandable dudit objet et un calculateur agencé pour déterminer un modèle additif définissant une forme d'objet à fabriquer et des données d'actionneurs pour l'objet à fabriquer.

Les travaux de la Demanderesse l'ont amenée à étudier la réalisation de structures présentant des propriétés de déformation anisotrope choisies et des propriétés déformables adaptées selon un cahier des charges fonctionnelles en général. Ainsi, elle a déposé une première demande de brevet français sous le numéro de demande FR 1652497 décrivant des mousses générées grâce à des cellules de Voronoï et présentant une élasticité variable dépendant de la structure retenue.

Rappelons qu'un diagramme de Voronoï s'applique à un ensemble de points d'un plan, ou d'un volume, appelés sites ou germes. Le diagramme est un réseau comportant des polygones convexes. Chacun délimite la zone dans laquelle tout point est plus proche de son germe que de tous les autres germes. Pour engendrer un diagramme de Voronoï seules certaines médiatrices sont utilisées et pas d'autres : les sites trop lointains ne sont pas pris en considération ; alors les segments utiles forment une triangulation ou partition du plan dite de Delaunay. On considère qu'en mécanique, une structure en triangulation de Delaunay minimise les carrés des aires des triangles. En comptant les frontières extérieures, chaque site est à l'intérieur d'un polygone convexe. Tous les points de ce polygone sont plus proches du point interne que de tous les autres points. Il s'agit d'un réseau en nid d'abeille irrégulier. À partir de ces fondements, il est possible de définir des segments (dans le plan) ou des surfaces bornées (dans l'espace). La connaissance de ces éléments discrets sert ensuite en fabrication additive pour réaliser un objet tridimensionnel ayant des propriétés mécaniques fonction de l'espace et des contraintes exercées sur l'objet.

Plus tard, la Demanderesse a déposé une deuxième demande de brevet français sous le numéro de demande FR1754866 permettant de réaliser des mousses orthotropes présentant des champs de déformation décrivant les directions dans lesquelles la mousse est plus susceptible de se déformer en réponse à une contrainte.

Bien qu'intéressantes d'un point de vue académique, ces inventions sont délicates d'utilisation dans des produits commerciaux, car leur déformabilité était difficile à mettre en action.

L'invention vient améliorer la situation. À cet effet, elle propose un dispositif pour fabrication additive selon la revendication 1.

Ce dispositif est particulièrement avantageux car il permet, par fabrication additive, de créer des objets dont la déformation est non seulement prévue, mais également commandable en présence d'une contrainte avantageusement mécanique.

Selon divers modes de réalisation, l'invention peut présenter une ou plusieurs des caractéristiques suivantes :
- les données d'actionneurs définissent les actionneurs pour l'objet à fabriquer en tant que structure additive, et dans lequel le calculateur est agencé pour déterminer un modèle additif définissant une mousse orthotropique qui intègre ces actionneurs,
- au moins un actionneur est réalisé au moyen d'une ou plusieurs structures fermées dans la mousse orthotropique, et par l'introduction dans celles-ci d'un gaz, d'un gel ou d'un fluide,
- l'actionneur agit par transition ci du gaz, du gel ou du fluide, suscitée au moyen d'une électrovanne, d'une source pneumatique, ou par simple pression sur l'une des parties renfermant le gaz, le gel ou le fluide en contact fluidique avec une autre partie de la mousse orthotropique propre,
- les données d'actionneurs définissent les actionneurs pour l'objet à fabriquer en tant qu'éléments non-additifs, et dans lequel le calculateur est agencé pour déterminer un modèle additif définissant une mousse orthotropique permettant l'introduction de ces actionneurs,
- les données d'actionneurs définissent les actionneurs pour l'objet à fabriquer comprend un élément déformable relié à divers points de la mousse orthotropique, de sorte que la déformation de l'élément déformable entraîne la déformation de la mousse orthotropique selon les directions privilégiées de déformation de chaque zone comprenant un point auquel est relié l'élément déformable, et
- le calculateur est agencé pour munir l'objet à fabriquer d'une peau sur tout ou partie de sa surface.

L'invention concerne également un procédé de détermination de données de modèle additif selon la revendication 8.

Elle concerne également un produit de programme d'ordinateur comprenant des portions de code de programme pour mettre en oeuvre le dispositif ou le procédé selon l'invention lorsque ledit programme est exécuté sur un ordinateur.

D'autres caractéristiques et avantages de l'invention apparaîtront mieux à la lecture de la description qui suit, tirée d'exemples donnés à titre illustratif et non limitatifs, tirés des dessins sur lesquels :
[Fig. 1] La figure 1 représente un premier exemple d'une mousse présentant des propriétés de déformation anisotrope choisies,
[Fig. 2] La figure 2 représente un exemple de mousse selon la Figure 1 munie d'un actionneur mécanique,
[Fig. 3] La figure 3 représente un deuxième exemple d'une mousse présentant un champ de déformations,
[Fig. 4] La figure 4 représente un exemple d'actionneur pouvant être introduit dans la mousse de la figure 1 ou la mousse de la figure 3, et
[Fig. 5] La figure 5 représente un exemple de diagramme schématique d'un dispositif selon l'invention.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

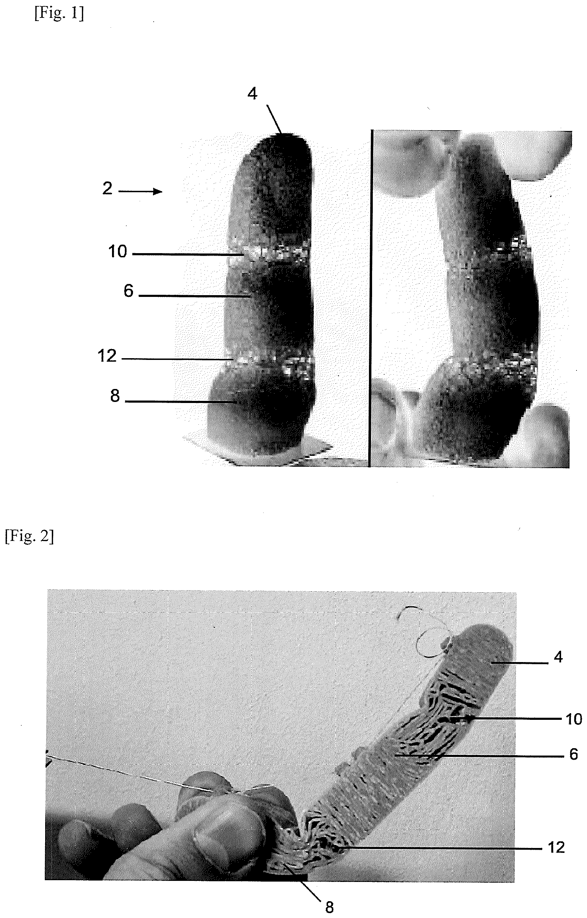

La figure 1 représente un premier exemple d'une mousse. Dans cet exemple, la mousse présente des propriétés de déformation anisotrope choisies. Comme on peut le voir sur cette figure, un objet 2 similaire à un doigt humain a été produit sous la forme d'une mousse présentant des propriétés de déformation élastique variable. Ainsi, l'objet 2 comprend une première portion d'extrémité 4, un élément médian 6 et une deuxième portion d'extrémité 8. La première portion d'extrémité 4 et l'élément médian 6 d'une part, et l'élément médian 6 et la deuxième portion d'extrémité 8 sont reliées entre elles respectivement par une première portion de jonction 10 et une deuxième portion de jonction 12.

Dans l'exemple décrit ici, cette mousse a été réalisée selon les enseignements de la demande FR1652497, c'est-à-dire qu'elle a été générée à l'aide de cellules de Voronoï et présente une densité moindre dans la première portion de jonction 10 et la deuxième portion de jonction 12 afin de créer des directions privilégiées de déformation lorsqu'une contrainte est appliquée sur la première portion d'extrémité 4 ou sur la deuxième portion d'extrémité 8. Ainsi, si l'on appuie sur l'une ou l'autre, le doigt se plie au niveau de ces portions, qui se comportent alors comme des phalanges.

La figure 2 représente une mousse selon l'invention qui tire avantage des propriétés de la mousse de la figure 1. En effet, un fil rigide a été attaché à une partie de la première portion d'extrémité 4, à l'élément médian 6, et à la deuxième portion d'extrémité 8.

Ainsi, en tirant sur une extrémité du fil tandis que l'une de la première portion d'extrémité 4 et de la deuxième portion d'extrémité 8 est fixe, le fil actionne le doigt pour le faire se replier au niveau de la première portion de jonction 10 et de la deuxième portion de jonction 12. Dans l'exemple décrit ici, le doigt n'a pas été muni d'une enveloppe formant peau.

Il peut néanmoins être avantageux de le prévoir. En effet, en l'absence de cette enveloppe, la structure peut s'écraser sous la tension du fil et sa déformation peut être plus difficile à maîtriser. En variante, tout type d'actionneur solide intégré à la mousse ou rapporté à celle-ci peut être utilisé, voire être formé en même temps que la mousse.

Lorsque la mousse orthotrope est enveloppée par un matériau de même nature ou de nature différente, cela permet de limiter les déformations spatiales dans des directions privilégiées, les exaltant relativement à une mousse non enveloppée. Ainsi, la mousse orthotrope est entourée d'une enveloppe externe imposant des déformations dans des directions privilégiées de l'espace. En l'absence de cette « peau », les effets pourront être moins notables parce qu'elle impose des contraintes de continuité spatiale qu'on ne retrouve pas dans une mousse non enveloppée,

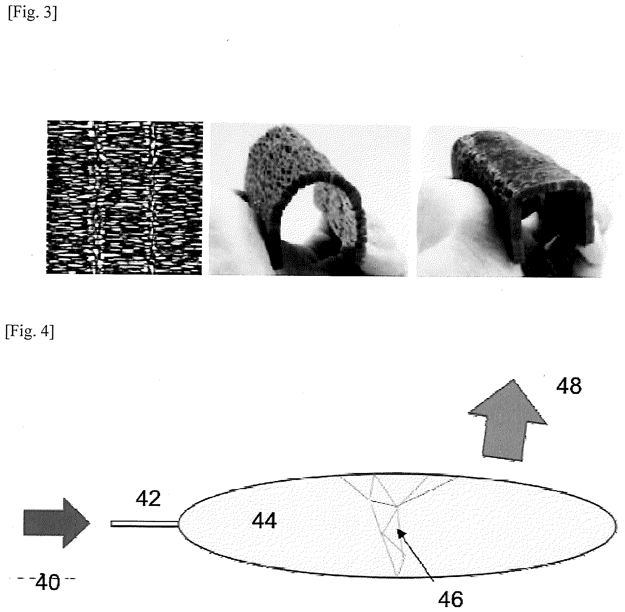

La figure 3 représente un premier exemple d'une mousse. Dans cet exemple, la mousse présente des propriétés orthotropes, et en particulier une carte de champs de déformations qui définit une pluralité de directions privilégiées lorsqu'une ou plusieurs contraintes sont appliquées. Cette mousse a été réalisée selon les enseignements de la demande FR1754866. D'une manière générale, les mousses fabriquées selon les enseignements de cette demande sont plus intéressantes que celles réalisées selon les enseignements de la demande FR1652497, car elle permet une plus grande richesse de directions privilégiées que cette dernière. Il est ainsi possible de réaliser des objets ayant une cinématique complexe.

Quand un matériau constitué d'une mousse homogène est plié, ou quand il est constitué de mousses orthotropes, les déformations peuvent être très différentes. C'est cette différence que l'on exploite dans la présente invention.

Afin de tirer avantage de cette structure, l'invention propose également des actionneurs du type de celui représenté sur la figure 4.

Dans l'exemple décrit ici, repose sur la réalisation d'une ou plusieurs structures fermées dans la mousse, et l'introduction dans celles-ci d'un gaz, d'un gel ou d'un fluide. L'idée ici est de faire transiter l'un de ces éléments d'une première partie de la mousse vers une deuxième partie de la mousse, qui se déformera selon ses directions privilégiées sous l'effet de la pression induite par l'excès de matière. La transition peut être suscitée avantageusement au moyen d'une électrovanne, d'une source pneumatique, ou par simple pression sur l'une des parties renfermant un gaz, un gel ou un fluide en contact fluidique avec une autre partie de la mousse propre à le recevoir. Par exemple, la pression exercée par le poids d'une personne sur un support placé par exemple dans une chaussure peut servir d'actionneur et entraîner des déformations souhaitées en termes de confort ou d'aide à la marche. Dans cet exemple, les raideurs introduites dans le ballonnet obligent une déformation dans des directions privilégiées.

Ainsi, sur l'exemple de la figure 4, une électrovanne introduit un gaz selon une direction 40 à travers un tuyau 42 dans une mousse orthotropique 44. La mousse orthotropique 44 comprend un réseau de tubes 46 qui sont reliés au tuyau 42, ainsi qu'une enveloppe en surface, de sorte que l'introduction du gaz va venir solliciter la mousse selon la direction 48.

En variante, les portions renfermant un gaz, un gel ou un fluide peuvent être entièrement fermées, de sorte qu'elles seules se déforment sous l'effet de la variation de pression interne. Néanmoins, les portions voisines étant elles-mêmes déformables, elles subiront au moins en partie une contrainte liée à la déformation de la portion sous pression. Dans ce cas le gaz, le gel ou tout autre fluide peuvent également être remplacés par un matériau à mémoire de forme que l'on active ou pas selon les besoins.

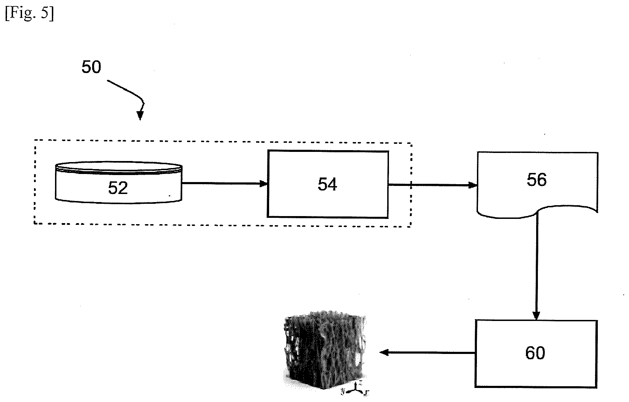

La figure 5 représente un exemple de mise en oeuvre schématique d'un dispositif selon l'invention.

Le dispositif 50 comprend une mémoire 52 qui reçoit des données définissant la forme générale à donner à un objet à fabriquer, ainsi que des données d'actionneurs qui définissent le type d'actionneur souhaité et leur emplacement dans l'objet.

Le dispositif comprend également un calculateur 54 agencé pour accéder aux données de la mémoire 52 et pour construire un modèle additif 56 pour réaliser la mousse ainsi que les éléments nécessaires pour que les actionneurs existent, c'est-à-dire les actionneurs eux-mêmes s'ils sont fabriqués de manière additive avec la mousse, ou les accès pour permettre leur installation ultérieure si nécessaire.

Enfin le dispositif 50 commande une imprimante 60 de fabrication additive, connue par ailleurs, pour réaliser la mousse selon le modèle additif déterminé par le calculateur 54.

## Revendications

1. Dispositif pour fabrication additive, comprenant
- une mémoire (52) agencée pour recevoir des données de forme d'objet à fabriquer et des données d'actionneurs pour l'objet à fabriquer, lesdites données d'actionneurs définissant des actionneurs configurés pour permettre une déformation prédéfinie et commandable dudit objet,
- le dispositif étant **caractérisé en ce qu'**il comprend
- un calculateur (54) agencé pour déterminer un modèle additif (56) définissant une mousse orthotropique à partir des données de forme d'objet à fabriquer et des données d'actionneurs pour l'objet à fabriquer.

2. Dispositif selon la revendication 1, dans lequel les données d'actionneurs définissent les actionneurs pour l'objet à fabriquer en tant que structure additive, et dans lequel le calculateur (54) est agencé pour déterminer un modèle additif (56) définissant une mousse orthotropique qui intègre ces actionneurs.

3. Dispositif selon la revendication 2, dans lequel les données d'actionneurs définissent au moins un actionneur prenant la forme d'une ou plusieurs structures fermées dans la mousse orthotropique, configurées pour recevoir un gaz, un gel ou un fluide.

4. Dispositif selon la revendication 3, dans lequel les données d'actionneurs définissent au moins un actionneur configuré pour agir par transition du gaz, du gel ou du fluide, suscitée au moyen d'une électrovanne, d'une source pneumatique, ou par simple pression, d'une première partie de la mousse orthotropique renfermant ledit gaz, ledit gel ou ledit fluide vers une deuxième partie de la mousse orthotropique avec laquelle elle est en contact fluidique.

5. Dispositif selon la revendication 1, dans lequel les données d'actionneurs définissent les actionneurs pour l'objet à fabriquer en tant qu'éléments non-additifs, et dans lequel le calculateur (54) est agencé pour déterminer un modèle additif (56) définissant une mousse orthotropique permettant l'introduction de ces actionneurs.

6. Dispositif selon la revendication 5, dans lequel les données d'actionneurs définissent les actionneurs pour l'objet à fabriquer comprend un élément déformable relié à divers points de la mousse orthotropique, de sorte que la déformation de l'élément déformable entraîne la déformation de la mousse orthotropique selon les directions privilégiées de déformation de chaque zone comprenant un point auquel est relié l'élément déformable.

7. Dispositif selon l'une des revendications précédentes, dans lequel le calculateur (54) est agencé pour munir l'objet à fabriquer d'une peau sur tout ou partie de sa surface.

8. Procédé de détermination de données de modèle additif comprenant :
a) recevoir des données de forme d'objet à fabriquer,
b) recevoir des données d'actionneurs pour l'objet à fabriquer, lesdites données d'actionneurs définissant des actionneurs configurés pour permettre une déformation prédéfinie et commandable dudit objet,
le procédé étant **caractérisé en ce qu'**il comprend :
c) déterminer un modèle additif (56) définissant une mousse orthotropique à partir des données de forme d'objet à fabriquer et des données d'actionneurs pour l'objet à fabriquer.

9. Produit de programme d'ordinateur comprenant des portions de code de programme pour mettre en oeuvre le procédé selon la revendication 8 lorsque ledit programme est exécuté sur un ordinateur.

## Patentansprüche

1. Vorrichtung zur additiven Fertigung, umfassend
- einen Speicher (52), der angeordnet ist, Formdaten eines herzustellenden Objekts und Aktorendaten für das herzustellende Objekt zu empfangen, wobei die Aktorendaten Aktoren definieren, die dazu ausgebildet sind, eine vorgegebene und steuerbare Verformung des Objekts zu ermöglichen,
- wobei die Vorrichtung **dadurch gekennzeichnet ist, dass** sie Folgendes umfasst einen Rechner (54), der angeordnet ist, ein additives Modell (56), das einen orthotropen Schaum definiert, anhand der Formdaten eines herzustellenden Objekts und der Aktorendaten für das herzustellende Objekt zu bestimmen.

2. Vorrichtung nach Anspruch 1, wobei die Aktorendaten die Aktoren für das herzustellende Objekt als additive Struktur definieren, und wobei der Rechner (54) angeordnet ist, ein additives Modell (56) zu bestimmen, das einen orthotropen Schaum definiert, der diese Aktoren enthält.

3. Vorrichtung nach Anspruch 2, wobei die Aktorendaten wenigstens einen Aktor definieren, der die Form einer oder mehrerer geschlossener Strukturen im orthotropen Schaum annimmt, die dazu ausgebildet sind, ein Gas, ein Gel oder ein Fluid aufzunehmen.

4. Vorrichtung nach Anspruch 3, wobei die Aktorendaten wenigstens einen Aktor definieren, der dazu ausgebildet ist, durch Übertritt des Gases, Gels oder Fluids, der mittels eines Magnetventils, einer Pneumatikquelle hervorgerufen wird, oder durch einfaches Drücken eines ersten Abschnitts des orthotropen Schaums, der das Gas, Gel oder Fluid einschließt, zu einem zweiten Abschnitt des orthotropen Schaums, mit dem sie in Kontakt steht, zu wirken.

5. Vorrichtung nach Anspruch 1, wobei die Aktorendaten die Aktoren für das herzustellende Objekt als nichtadditive Elemente definieren, und wobei der Rechner (54) angeordnet ist, ein additives Modell (56) zu bestimmen, das einen orthotropen Schaum definiert, der die Einführung dieser Aktoren ermöglicht.

6. Vorrichtung nach Anspruch 5, wobei die Aktorendaten die Aktoren für das herzustellende Objekt definieren, das ein verformbares Element umfasst, das mit verschiedenen Punkten des orthotropen Schaums verbunden ist, so dass die Verformung des verformbaren Elements die Verformung des orthotropen Schaums in den bevorzugten Verformungsrichtungen jedes Bereichs herbeiführt, der einen Punkt umfasst, mit dem das verformbare Element verbunden ist.

7. Vorrichtung nach einem der vorhergehenden Ansprüche, wobei der Rechner (54) angeordnet ist, das herzustellende Objekt mit einer Haut auf dessen gesamten Oberfläche oder einem Abschnitt davon zu versehen.

8. Verfahren zur Bestimmung von Daten eines additiven Modells umfassend:
a) Empfangen der Formdaten eines herzustellenden Objekts,
b) Empfangen der Aktorendaten für das herzustellende Objekt, wobei die Aktorendaten Aktoren definieren, die dazu ausgebildet sind, eine vorgegebene und steuerbare Verformung des Objekts zu ermöglichen,
wobei das Verfahren **dadurch gekennzeichnet ist, dass** es Folgendes umfasst:
c) Bestimmen eines additiven Modells (56), das einen orthotropen Schaum definiert, anhand der Formdaten eines herzustellenden Objekts und der Aktorendaten für das herzustellende Objekt.

9. Computerprogrammprodukt umfassend Programmcodeabschnitte zur Durchführung des Verfahrens nach Anspruch 8, wenn das Programm auf einem Computer ausgeführt wird.

## Claims

1. Device for additive manufacturing, comprising:
- a memory (52) arranged to receive shape data of object to be manufactured and actuator data for the object to be manufactured, said actuator data defining actuators configured to enable a predefined and controllable deformation of said object,
- said device being **characterized in that** it comprises a computer (54) arranged to determine an additive model (56) defining an orthotropic foam using the shape data of the object to be manufactured and actuator data for the object to be manufactured.

2. Device according to claim 1, wherein the actuator data define the actuators for the object to be manufactured as an additive structure, and wherein the computer (54) is arranged to determine an additive model (56) defining an orthotropic foam which integrates these actuators.

3. Device according to claim 2, wherein the actuator data define at least one actuator in the form of one or more closed structures in the orthotropic foam, configured to receive a gas, a gel or a fluid therein.

4. Device according to claim 3, wherein the actuator data define at least one actuator configured to act by transition of the gas, gel or fluid, triggered by means of a solenoid valve, a pneumatic source, or by simple pressure, from a first part of the orthotropic foam containing said gas, said gel or said fluid to a second part of the orthotropic foam with which it is in fluidic contact.

5. Device according to claim 1, wherein the actuator data define the actuators for the object to be manufactured as non-additive elements, and wherein the computer (54) is arranged to determine an additive model (56) defining an orthotropic foam enabling the introduction of these actuators.

6. Device according to claim 5, wherein the actuator data define the actuators so that the object to the manufactured comprises a deformable element connected to various points of the orthotropic foam, such that the deformation of the deformable element induces the deformation of the orthotropic foam according to the preferred directions of deformation of each zone comprising a point to which the deformable element is connected.

7. Device according to one of the preceding claims, wherein the computer (54) is arranged to provide the object to be manufactured with a skin on all or part of the surface thereof.

8. Method for determining additive model data comprising:
a) receiving shape data of object to be manufactured,
b) receiving actuator data for the object to be manufactured, said actuator data defining actuators configured to enable a predefined and controllable deformation of said object,
said method being **characterized in that** it comprises:
c) determining an additive model (56) defining an orthotropic foam from the shape data of the object to be manufactured and the actuator data for the object to be manufactured.

9. Computer program product comprising portions of program code to implement the method according to claim 8 when said program is run on a computer.
